# FASCICULE DE BREVET EUROPEEN

(11) **EP 2 627 736 B1**
(45) Date de publication et mention de la délivrance du brevet: **04.12.2019**
(21) Numéro de dépôt: 11832095.1
(22) Date de dépôt: 11.10.2011
(51) Int. Cl.: C10G 11/18, B01J 8/24, B01J 38/06, B01J 38/56, C07C 11/04, C07C 11/06, B01J 8/28, C07C 11/02, B01J 38/14, C07C 4/06, C10G 51/02

(54) **PROCEDE DE CRAQUAGE ET DE STRIPAGE MULTI-ETAGE DANS UNE UNITE DE FCC.**
MEHRSTUFIGES RISSBILDUNGS- UND ABLÖSUNGSVERFAHREN IN EINER FCC-EINHEIT
MULTISTAGE CRACKING AND STRIPPING PROCESS IN AN FCC UNIT

(30) Priorité: 15.10.2010 FR 1058432
(43) Date de publication de la demande: 21.08.2013
(73) Titulaire: Total Raffinage France, 92400 Courbevoie (FR)
(72) Inventeur: LEROY, Patrick, F-76430 Saint Vigor D' ymonville (FR); BORIES, Marc, F-76280 Saint Jouin De Bruneval (FR); ECHARD, Michaël, F-76290 Montivilliers (FR)
(74) Mandataire: Raboin, Jean-Christophe
(86) Numéro de dépôt international: PCT/FR2011/052366
(87) Numéro de publication internationale: WO 2012/049416

(56) Documents cités:
- EP-A1- 1 577 368
- EP-A1- 2 072 605
- EP-A1- 2 113 299
- EP-A2- 0 850 687
- US-A1- 2005 040 075
- US-A1- 2006 021 909
- CORMA A ET AL: "Different process schemes for converting light straight run and fluid catalytic cracking naphthas in a FCC unit for maximum propylene production", APPLIED CATALYSIS A: GENERAL, ELSEVIER SCIENCE, AMSTERDAM, NL, vol. 265, no. 2, 8 juillet 2004 (2004-07-08), pages 195-206, XP004508848, ISSN: 0926-860X, DOI: DOI:10.1016/J.APCATA.2004.01.020 cité dans la demande

## Description

La présente invention concerne un procédé de stripage multi-étagé utilisé dans un procédé de craquage catalytique à lit fluidisé ou FCC (Fluidized catalytic cracking) pour la maximisation de la production d'oléfines, c'est-à-dire d'oléfines en C₃ et C₄, notamment de propylène. Ce procédé, s'intégrant dans le procédé de craquage catalytique, participe aux processus limitant la production d'essence dont la production est excédentaire notamment en Europe, et contribue de ce fait non seulement à augmenter la production d'oléfines mais aussi à maximiser la production de bases gazoles. Il s'applique à tout procédé de FCC comprenant une étape de réaction dans un réacteur à lit fluidisé ascendant ou descendant, une étape de séparation et de stripage des hydrocarbures craqués et des grains de catalyseur cokés et une étape de régénération du catalyseur. Elle concerne également les dispositifs de mise en oeuvre du dit procédé, utilisables dans une unité de craquage catalytique, celle-ci pouvant comprendre un ou plusieurs réacteurs ascendants et/ou descendants.

On entend ici par stripage, l'opération qui consiste à extraire au moyen d'un fluide gazeux, les hydrocarbures piégés dans les grains de catalyseur à la sortie du réacteur fluidisé. De plus, la capacité de l'unité de FCC dans laquelle cette opération est réalisée est appelée désengageur/stripeur. On appellera aussi par la suite désengageur, la partie du dit désengageur/stripeur permettant de séparer les grains des hydrocarbures par séparation balistique ou centrifuge, et stripeur la partie de celui-ci dans laquelle le stripage des grains est effectué.

Depuis de longues années les raffineurs et les sociétés bailleur de licences des technologies FCC travaillent à l'optimisation du fonctionnement de ces procédés et des unités correspondantes. L'optimisation des procédés FCC a tout d'abord été dirigée vers la production de produits légers de type gaz liquéfiés, naphtas et essences, ces produits correspondant à la demande du marché. Il y avait essentiellement une demande d'oléfines légères de C₂ à C₄, par les fabricants de polymères et une demande en essences principalement pour la consommation du parc automobile véhicules légers, la demande en bases gazoles n'étant pas majoritaire.

Pour rappel, lors d'une opération de craquage catalytique, la charge est injectée dans un réacteur principal où elle est mise en contact avec des grains de catalyseur chauds en lit fluidisé, provenant du régénérateur chargé de brûler sous atmosphère oxydante le coke déposé sur les grains de catalyseurs lors du craquage de la charge. En fin de réacteur, les hydrocarbures craqués sous forme gazeuse sont séparés des grains de catalyseur cokés dans le désengageur/stripeur. A la sortie du désengageur de ce désengageur/stripeur, les hydrocarbures craqués sous forme gazeuse sont dirigés vers une unité de fractionnement pour y être divisés en plusieurs coupes hydrocarbonées. Simultanément, les grains de catalyseur cokés sont récupérés dans le stripeur du désengageur/stripeur sous forme d'un lit dense où ils sont balayés à contre courant par un gaz inerte, préférentiellement de la vapeur, dont la fonction est d'éliminer toutes traces d'hydrocarbures résiduels piégés dans les pores de chaque grain de catalyseur ou dans les interstices du lit dense. En sortie du stripeur, les grains de catalyseur cokés et stripés sont dirigés vers le régénérateur.

Généralement, en fonctionnement stabilisé, sans contrainte extérieure, c'est-à-dire dans des conditions stables de température, pression et vitesse de circulation du catalyseur dans l'unité, on obtient un équilibre thermique lorsque la quantité de coke formée reste constante dans le réacteur. On caractérise cette quantité de coke formée et nécessaire à la stabilité du fonctionnement de l'unité par le « DELTA coke ». Ce Delta coke correspond à la différence entre la quantité de coke présente à l'entrée de la zone de régénération et celle présente sur le catalyseur régénéré en sortie de cette zone. Si le Delta coke et donc la température du catalyseur régénéré augmentent du fait de la présence d'hydrocarbures encore piégés dans les grains de catalyseur, il sera nécessaire modifier les paramètres de réglage de l'unité. Par exemple, on pourra réduire le débit des grains de catalyseur circulant pour maintenir la température de réaction de la charge à craquer dans des limites acceptables ou encore ajuster la préchauffe de la charge.

Aussi, les ingénieurs chargés d'améliorer le fonctionnement du FCC se sont intéressés particulièrement à la problématique du stripage optimal des grains cokés de catalyseurs récupérés en sortie de réacteur et de la configuration possible des désengageurs/stripeurs. En effet, il est essentiel de séparer le plus efficacement possible les hydrocarbures piégés dans les grains de catalyseurs cokés. Un stripage insuffisant des grains cokés est à la source d'une combustion plus importante au sein du régénérateur, donc d'une augmentation de la production de chaleur transportée par le catalyseur dans le réacteur et enfin à une augmentation des gaz secs non désirés et une perte de production en produits convertis attendus tels que les oléfines de C₃ à C₄, les essences et les bases gazoles. En outre, une surchauffe du régénérateur pourrait causer des dommages métallurgiques dans les cas extrêmes, et une désactivation accélérée du catalyseur. Par contre, un stripage trop long peut être à l'origine de réactions secondaires non désirées telle qu'une cokéfaction particulièrement excessive des grains de catalyseur risquant de conduire à terme à une désactivation partielle de ceux-ci. Le temps de stripage doit donc être optimal pour éviter ces deux écueils et maintenir des conditions stables de fonctionnement de l'unité de FCC.

L'efficacité de l'opération de stripage est essentielle pour le bon contrôle des opérations de craquage. Cette efficacité se mesure par détermination de l'hydrogène résiduel dans le coke des grains de catalyseur. On parle de bonne efficacité de stripage lorsque cette valeur est comprise entre 6 et 6.5% poids par rapport au poids de coke.

Pour atteindre, voire améliorer cette efficacité du stripage, les professionnels des procédés FCC ont développé de nombreuses technologies permettant de favoriser cette séparation. D'une manière générale, il s'est avéré que l'introduction de chicanes dans le stripeur sur le parcours du lit dense de grains de catalyseur vers le régénérateur tout en maintenant le balayage à contre-courant de ces grains par un fluide gazeux inerte, favorisait la séparation recherchée.

Parallèlement à la mise en oeuvre de ces chicanes, les professionnels ont développé des garnissages susceptibles de mieux diviser le lit dense de grains de catalyseur cokés en une multitude de flux et de rendre ces grains plus accessibles au fluide de stripage et ainsi d'éliminer davantage d'hydrocarbures piégés dans les grains par exemple selon le brevet WO01/047630.

La demanderesse, dans son brevet EP719850, a décrit un garnissage structuré, encore appelé packing en anglais, destiné à être disposé dans le stripeur au niveau de la formation du lit dense de catalyseur retournant vers le régénérateur. Ce garnissage comprend au moins un élément, disposé sur toute la section transversale du stripeur à ce niveau : cet élément comporte des cellules juxtaposées s'orientant de façon sensiblement radiale afin de diviser le lit dense en un grand nombre de flux ce qui permet ainsi le passage simultané des grains de catalyseur et du fluide de stripage. Plus précisément, la demanderesse a recommandé l'utilisation de tôles plissées disposées les unes sur les autres de telle façon que les plis respectifs de deux tôles accolées fassent un angle variant de 10 à 90°.

Depuis d'autres garnissages ont été proposés. Par exemple dans les brevets US7022221, US7077997 et WO2007/094771, on propose des déflecteurs présentant la forme de plateaux disposés dans le stripeur autour du riser, chaque plateau comprenant une multitude d'orifices déterminés par des grilles ou des trous formés présentant des parois en déclivité par rapport au plan du dit plateau. Ces orifices laissent passer les grains et le gaz de stripage injecté à contre-courant par rapport au flux des grains de catalyseur depuis au moins un point situé en aval des déflecteurs.

Dans le brevet WO00/35575, on propose l'entrecroisement de paires de plaquettes de sections métalliques planes pour créer un garnissage de la partie stripeur du FCC afin de diviser le flux de catalyseur coké comme précédemment avec envoi d'un flux gazeux à contre-courant injecté en aval de cet entrecroisement.

Dans le brevet chinois publié CN1763150, plusieurs plateaux comprenant un agencement régulier d'ailettes de guidage perpendiculaires au plan de chacun des plateaux, sont disposés dans le stripeur les uns sur les autres. Les plateaux vicinaux sont placés pour que les ailettes du premier soient perpendiculaires à celles du second et ainsi de suite de façon à déterminer des chemins préférentiels permettant la division et le stripage des grains de catalyseur par le fluide de stripage injecté en aval des plateaux à contre-courant.

Dans le brevet EP1577368, il s'agit d'intégrer autour du riser à l'intérieur du stripeur un garnissage constitué de plusieurs couches superposées, chacune d'elles étant constituée d'une pluralité de rubans ondulés juxtaposés. De préférence, deux couches voisines sont disposées de telle façon à ce que les rubans de la première couche et ceux de la deuxième couche fassent un angle non nul. Les monts, vallées et orifices d'un plateau pris en combinaison avec les monts, vallées et orifices des plateaux disposés de part et d'autre déterminent un chemin dans le garnissage ainsi structuré permettant la circulation du flux des grains de catalyseur et leur stripage par un fluide de stripage injecté en aval du dit garnissage.

Dans tous ces garnissages, l'objectif est d'optimiser le stripage du catalyseur mais aucun ne constitue un moyen de réduire la production d'hydrocarbures non désirés, objectif de la présente invention.

Dans un contexte de maximisation de la production d'oléfines légères et de minimisation des essences produites au FCC, la Demanderesse se propose de recycler des hydrocarbures non désirés récupérés au fractionnement, au niveau du stripeur de FCC afin de craquer ceux-ci.

Malgré la réduction de l'activité catalytique du solide liée à la présence de coke, le professeur Corma a montré (Corma et al., Applied Catalysis A: General 265 (2004) 195-206) que le niveau de conversion d'une coupe hydrocarbonée légère, par exemple une essence catalytique, en lit dense dans des conditions de craquage telles que rencontrées dans un stripeur s'est révélé intéressant avec notamment la production de propylène (oléfine en C₃) avec peu de coke et de gaz secs.

Cette technologie a déjà été envisagée par le brevet US7658837 qui propose l'injection homogène d'un ou plusieurs flux d'hydrocarbures, par exemple de naphta ou de LCO (light cycle oil, soit en français gazole léger de craquage catalytique) d'origine FCC ou issu d'un cokeur, permettant soit de réduire le soufre et d'améliorer la qualité des produits soit de favoriser la production d'oléfines légères, notamment de type propylène.

L'utilisation de ce type de recycle, mais avec des hydrocarbures plus cokant, a aussi été décrit dans le document EP2072605 pour augmenter la quantité de coke sur le catalyseur envoyé au régénérateur, l'objectif de cet ajout de coke étant de faire fonctionner le régénérateur comme un gazéifieur, susceptible de produire du gaz de synthèse, en injectant dans le régénérateur non plus de l'oxygène mais un mélange oxygène, vapeur d'eau et gaz carbonique CO₂, recueilli sur les autres unités environnantes.

Dans ces deux documents, les hydrocarbures sont craqués en une seule étape au stripeur, de la vapeur d'eau de stripage étant injecté après l'étape de craquage, mais aussi avant dans le document EP2072605.

Le but de la présente invention est de transformer l'étape d'un procédé FCC utilisée exclusivement pour séparer et stripper les grains de catalyseur et limiter l'entraînement d'hydrocarbures vers le régénérateur, en au moins deux étapes de réaction (craquage) et au moins deux étapes de stripage permettant d'améliorer les performances économiques du procédé FCC sans modifier l'efficacité du stripage fonction initiale d'une telle étape.

La demanderesse a donc développé un nouveau procédé de réaction et de stripage qui combine le multi-craquage d'hydrocarbures sur les grains de catalyseur cokés, notamment d'hydrocarbures dont on veut minimiser la production, et le stripage de ces grains de catalyseurs cokés en présence d'un garnissage particulier notamment dans le stripeur où a lieu ce stripage. Il s'agit ici d'utiliser le stripeur ainsi modifié, comme une multi- zone de craquage, c'est-à-dire à plusieurs étapes de craquage, dans laquelle il est possible par exemple, de recycler des hydrocarbures non désirés dont on veut diminuer la production, obtenus par craquage au niveau du réacteur principal. La demanderesse envisage cette combinaison aussi bien pour le remodelage d'unités existantes que pour la construction d'unités nouvelles.

La présente invention vise à augmenter la flexibilité de fonctionnement des unités FCC en fonction des marchés. Cette flexibilité est atteinte en minimisant la production d'hydrocarbures non désirés et en maximisant la production d'hydrocarbures à haute valeur ajoutée tels que les oléfines légères en C₃ et C₄ et/ou les distillats de type gazole par la mise en oeuvre d'un nouveau procédé de réaction et de stripage multi-étagé facilement intégrable dans un procédé de craquage catalytique classique.

On entend ici par procédé de craquage catalytique classique un procédé comprenant une étape de réaction d'une charge hydrocarbonée sur un lit fluidisé de catalyseur ascendant ou descendant, une étape de désengagement/ stripage des grains de catalyseur cokés d'avec les effluents craqués,(c'est-à-dire séparation des grains des effluents puis stripage de ces grains), et enfin une étape de régénération, en un ou plusieurs étages, des grains de catalyseurs cokés, les grains de catalyseur régénérés récupérés en sortie, étant renvoyés à l'entrée du lit fluidisé de l'étape de réaction.

La présente invention a donc pour objet un procédé de craquage catalytique classique à lit fluidisé selon la revendication 1 comprenant un procédé de craquage et de stripage multi-étagé d'un mélange fluidisé d'hydrocarbures et de grains de catalyseur cokés, intégré dans une étape de désengagement/ stripage du procédé de craquage catalytique classique à lit fluidisé, ledit procédé multi-étagé comprenant au moins une étape de craquage et une étape de stripage après séparation des grains de catalyseur cokés et des effluents craqués, caractérisé en ce que le dit procédé de craquage et de stripage multi-étagé comprend au moins deux étapes de craquage d'au moins un fluide hydrocarboné sur les grains de catalyseur cokés séparés, suivies d'au moins deux étapes de stripage de ces grains, chaque étape de craquage précédant une étape de stripage.

Dans le cadre de la présente invention, le fluide hydrocarboné est injecté sur les grains de catalyseur cokés au moyen d'un ou plusieurs injecteurs au cours de la première étape de craquage, tandis que le stripage des grains de catalyseurs cokés est effectué au cours de l'étape de stripage, par un fluide de stripage, injecté à contre-courant des grains de catalyseur utilisés à l'étape précédente.

Parmi les avantages de l'invention, le premier avantage est de créer au moins deux zones de réaction supplémentaires en plus du (ou des) réacteur(s) ascendant(s) ou descendant(s) dans la même unité FCC : elle permet de consommer des hydrocarbures dont on veut limiter la production sans limiter la capacité hydraulique de l'unité, tout en assurant un stripage efficace du catalyseur en sortie du stripeur afin de maintenir des équilibres thermiques de l'unité de FCC.

Dans un mode préféré de l'invention, le procédé selon l'invention, intégré dans l'étape de désengagement/stripage d'une unité de craquage catalytique classique, comprendra avantageusement en amont de la première étape de craquage, une étape de pré-stripage du catalyseur consistant à balayer à contre-courant du flux des grains de catalyseur cokés (issus de la séparation effluents craqués/catalyseur coké) par un fluide de stripage injecté à contre-courant du dit flux ; le débit de ce fluide varie préférentiellement de 10 à 40% du débit total de fluide utilisé pour le stripage des grains cokés dans le dit procédé selon l'invention.

L'ajout d'une phase de pré-stripage des grains de catalyseur constitue le deuxième avantage de l'invention : cette phase permet de restaurer une partie de l'activité du catalyseur en éliminant (au moins partiellement) les hydrocarbures restant piégés et adsorbés dans les grains après la séparation, avant la première étape de craquage et donc de maximiser la conversion de la coupe hydrocarbonée injectée au cours de la première étape de craquage.

On distribue les grains dans les différents compartiments dédiés au craquage et éventuellement au stripage au moyen d'au moins un élément de garnissage structuré de type packing décrit notamment dans les brevets cités dans la présente description.

Ces éléments de garnissage structurés permettent une distribution parfaitement homogène des grains de solide sur toute la section du stripeur. L'écoulement du solide est alors assimilable à un écoulement de type piston sans rétro-mélange, améliorant le contact entre les phases solide et gazeuse sans passage préférentiel du gaz. De plus, les bulles de gaz montantes, dont le volume a tendance à augmenter par coalescence de bulles plus petites, sont alors cassées lors du passage dans de tels garnissages structurés, ce qui favorise le transfert de matière entre la phase gaz et la phase solide.

En l'absence de tels éléments de garnissage, le volume des bulles de gaz limiterait ainsi leur temps de séjour dans le stripeur en raison de leur vitesse ascensionnelle trop élevée. L'échange de matière avec le solide descendant serait alors fortement limité en raison du faible temps de séjour des bulles dans le stripeur mais également en raison de la diminution de la surface d'échange entre les deux phases, les bulles étant trop grandes.

De plus les conditions de craquage, au sens catalytique du terme sont très favorables, avec un ratio (C/O) catalyseur sur hydrocarbures extrêmement élevé, compris entre 30 et 200, et une température de réaction légèrement inférieure à celle mesurée en sortie du réacteur de l'ordre de 1 à 5°C.

Un avantage de la présente invention est aussi qu'elle permet de créer les conditions hydrauliques, catalytiques et thermiques optimales pour limiter la production de coke et de gaz secs au profit d'oléfines légères (C₃ à C₄) bien que l'activité du catalyseur utilisé soit réduite par le coke résiduel présent. Cette baisse d'activité catalytique est en grande partie compensée par le ratio catalyseur sur hydrocarbures très élevé.

Cette étape de pré-stripage est tout à fait similaire à celle pratiquée par l'homme du métier via l'utilisation d'éléments de garnissage structurés pour le stripage des hydrocarbures encore présents dans les grains de catalyseur avant l'étape de régénération.

Les étapes de stripage du dit procédé selon l'invention, comprennent au moins une injection de gaz constituée majoritairement, par exemple au moins 60% (en volume), de vapeur d'eau.

Les étapes de craquage comprennent au moins une injection d'hydrocarbures, de préférence une coupe hydrocarbonée recyclée, pouvant contenir moins de 5% (en volume) de vapeur d'eau, notamment pour aider à l'atomisation et la vaporisation des hydrocarbures les plus lourds, notamment des distillats de type gazole. Par exemple, la coupe hydrocarbonée injectée comprendra avantageusement de 1 à 3% (en volume) de vapeur d'eau.

Un autre effet technique de l'invention est d'obtenir un craquage plus sélectif au cours des étapes de craquage pour maximiser la production d'un produit désiré au cours de l'étape de désengagement/ stripage. Comparativement au craquage dans le réacteur, le craquage dans l'étape de désengagement/ stripage se fait dans des conditions telles que le rapport débit de catalyseur sur débit de charge fraîche (C/O) est élevé et la température plus basse, ce qui favorise les réactions catalytiques par rapport aux réactions thermiques, ces dernières générant des produits non désirés comme les gaz secs. L'utilisation de recycle d'hydrocarbures dans le stripeur permet alors de réduire la sévérité des conditions de craquage dans le réacteur (typiquement en baissant de la température de réaction). Principal. Ainsi, le craquage d'une essence catalytique en lit dense fluidisé conduit sélectivement à la production de gaz de pétrole liquéfié ou GPL, qui permettra à iso production totale de GPL de baisser la température de réaction (TRX ou ROT) du réacteur principal de craquage de la charge. En diminuant cette température, la production de gaz secs et autres produits secondaires comme les di-oléfines sera réduite, soulageant ainsi le compresseur de gaz craqués disposé en tête de colonne de fractionnement des effluents craqués. Ce gain en volume pourra être mis à profit en craquant davantage d'essences dans le stripeur et/ou en utilisant davantage d'additif de conversion type ZSM5, pour augmenter la production de GPL tant au niveau de la zone de craquage principale de la charge que dans les étapes de craquage de la zone de stripage.

Un autre effet technique de la présente invention est de pouvoir bénéficier d'un effet catcooler (ou refroidissement du catalyseur) indépendant de la ROT ou TRX. Si la production de coke reste faible, la coupe hydrocarbonée introduite en aval d'une étape de craquage va refroidir les grains de catalyseur. Par exemple, pour une unité dont le débit de circulation est de 40 tonnes/minute (t/min), l'introduction de 20 tonnes/heure (t/h) d'essence au cours d'une phase de craquage conduira à diminuer la température des grains de catalyseur de 5°C, cette diminution de température étant également constatée dans la phase dense du catalyseur présent dans le régénérateur.

Dans le procédé selon l'invention, deux étapes de craquage s'intercalant entre deux étapes de stripage favorisent donc la conversion d'une coupe hydrocarbonée de température d'ébullition typiquement inférieure à 220°C en hydrocarbures légers type GPL, sans modification de l'équilibre hydraulique du procédé de craquage catalytique dans lequel il s'intègre, en augmentant faiblement la quantité de coke présente sur les grains de catalyseur.

Dans un procédé de FCC fonctionnant en mode maximisation de distillats, notamment de type gazole, le recycle comprendrait avantageusement l'essence produite ou tout fluide de température d'ébullition inférieure ou égale à 220°C et/ou encore des produits de type HCO (heavy cycle oil ou encore gazole lourd aromatique) ou des slurry (ou boues hydrocarbonées résiduelles) récupérées au niveau de l'étape de fractionnement des hydrocarbures du procédé FCC. Dans ce mode de maximisation du distillat, on préférera travailler à basse conversion avec une température de réaction au niveau du réacteur principal variant typiquement de 490 à 510°C, se traduisant par des rapports débit de catalyseur sur débit de charge fraiche (C/O) variant de 4 à 6. On peut également introduire dans le procédé de l'invention, des hydrocarbures provenant d'autres unités de raffinage (coker, hydrocraqueur, viscoréducteurs, distillation atmosphérique etc..) ou même des produits issus de la biomasse, par exemple des huiles végétales ou des hydrocabures verts issus de la biomasse de première ou deuxième génération. On ne sortirait pas du cadre de l'invention, si on introduisait simultanément des hydrocarbures en combinaison avec des huiles végétales telles que les huiles de colza ou de palme.

Dans un mode préféré de l'invention, les hydrocarbures pour le recycle peuvent être choisis parmi les oléfines en C₆ et C₇ et les oligomères de température d'ébullition inférieure à 220°C résultant de la polymérisation des oléfines de C₂ à C₅.

Les différents hydrocarbures décrits ci-dessus peuvent être introduits seuls ou en mélange.

Ainsi, il est possible d'injecter des hydrocarbures de même nature dans des étapes de craquage différentes ou des hydrocarbures de nature différente à chacune des étapes de craquage ou encore des hydrocarbures de nature différente conjointement dans une même étape de craquage.

Pour atteindre l'objectif commun de maximisation des distillats et d'optimisation de la production d'oléfines en C₃ et C₄ et plus particulièrement en propylène, on injecte dans la première étape de craquage au moins un hydrocarbure choisi parmi l'essence produite ou tout fluide de température d'ébullition inférieure ou égale à 220°C, préférentiellement inférieur ou égale à 160°C, et dans la deuxième étape de craquage des produits de type HCO ou du slurry, récupérés au niveau de l'étape de fractionnement des hydrocarbures du procédé de craquage catalytique à lit fluidisé, ces deux étapes étant séparées par une étape de stripage. Dans une telle configuration le ROT ou TRX sera choisie de préférence entre 490 et 510°C, résultant d'un rapport débit de catalyseur sur débit de charge fraîche (C/O) compris entre 4 et 6 permettant de minimiser la conversion dans le riser principal, typiquement comprise entre 60-65%pds,

Dans un autre mode de réalisation préféré, le procédé selon l'invention comprendra une succession d'étapes de craquage et de stripage, comprenant éventuellement une étape initiale de pré-stripage. Il est possible d'introduire autant d'étapes de craquage et de stripage que l'on souhaite, la dernière étape étant toujours une étape de stripage en vue de préparer les grains de catalyseur à être régénérés, cette étape réduisant l'entraînement d'hydrocarbures susceptibles d'augmenter la température du régénérateur.

Une étape de stripage comprendra avantageusement au moins un élément de garnissage structuré et un système d'injection de vapeur à contrecourant du flux de catalyseur. Parallèlement, une étape de craquage comprendra un système d'injection d'hydrocarbures et éventuellement au moins un élément de garnissage structuré dans tout ou partie du volume dédié au craquage.

On ajustera l'agencement et les conditions opératoires du procédé de l'invention de manière à respecter les règles appliquées communément par l'homme du métier, notamment la vitesse ascensionnelle des gaz, le flux solide descendant et le temps de résidence en particulier au niveau de l'étape de stripage avant la rentrée du catalyseur dans le régénérateur.

Dans le procédé de craquage catalytique intégrant le procédé de l'invention, le catalyseur est un catalyseur conventionnel dédié à la production d'essence à partir de charge lourde type résidu atmosphérique (surface totale de préférence supérieure ou égale à 110 m²/g et dont la teneur en métaux contaminants, Nickel et Vanadium, de préférence inférieure à 10000 ppm poids pour limiter les effets secondaires indésirables comme la production de gaz secs (c'est-à-dire notamment la production d'éthylène, de méthane, d'hydrogène et H₂S) et de coke. Dans un mode préféré de l'invention, on peut ajouter à la masse catalytique constituée par ce catalyseur conventionnel en tout ou partie coké dans certaines phases du procédé, jusqu'à 15% en poids d'additif, par exemple d'additif ZSM5, favorisant la formation d'oléfines par craquage des essences, qu'elles soient issues du craquage de la charge injectée dans le réacteur ascendant ou du recycle d'hydrocarbures dans le stripeur du désengageur stripeur de l'unité de craquage catalytique. Dans la pratique, au-delà de la concentration de 15% poids d'additif, on observe un phénomène de dilution de l'activité catalytique du catalyseur assurant le craquage de la charge, ce qui limite la production de molécules d'essence et par voie de conséquence, la production en oléfines légères de C2 à C4. L'appoint d'additif peut se faire indépendamment de l'appoint de catalyseur, en mélange avec celui-ci dans le rapport approprié.

La présente invention a également pour objet un procédé selon la revendication 10 qui met en œuvre un dispositif de craquage et de stripage adapté aux unités de craquage catalytique placé dans une capacité dite stripeur du désengageur/stripeur(7) disposée en aval du réacteur principal contenant le lit fluidisé, cette capacité comprenant un désengageur pour la séparation centrifuge et/ou balistique des grains de catalyseur et des hydrocarbures craqués issus du réacteur principal, et un stripeur pour le stripage des grains de catalyseur (9) disposé en amont de la sortie (8) du catalyseur vers le régénérateur et le stripeur comprend au moins quatre compartiments, dont au moins deux compartiments de craquage (2) et au moins deux compartiments de stripage (3), équipés d'éléments de garnissage structurés ou packing, ces éléments occupant tout ou partie de la section du dit compartiment, et chaque compartiment comportant à sa base au moins un moyen d'injection de fluides gazeux (5), par exemple au moyen d'une couronne d'injection et/ou d'injecteurs.

Dans un mode préféré de l'invention, le dispositif comprend en sus au moins un compartiment de pré-stripage (1) des grains de catalyseur situé en amont du compartiment de craquage (2), avantageusement équipé d'au moins un injecteur (3) de fluide de stripage et éventuellement d'au moins un garnissage structuré ou non.

De préférence, le dispositif de l'invention comprend une pluralité de compartiments de craquage et de stripage intercalés, équipés d'au moins un moyen d'injection de fluides gazeux, les volumes de la totalité des étapes de craquage et de stripage correspondant respectivement de 25 à 65% et de 35 à 75% du volume total occupé. S'il y a un compartiment de pré-stripage, celui-ci pourrait occuper jusqu'à 25% du volume dédié au stripage.

Selon l'invention les compartiments de craquage et de stripage sont équipés d'éléments de garnissage structurés ou packing tels que décrits précédemment dans les brevets cités dans la présente description. Plus particulièrement, dans les compartiments de craquage du dispositif selon l'invention, les éléments de garnissage occuperont tout ou partie de la section du compartiment. On ne sortirait pas ainsi du cadre de la présente invention si sur toute la hauteur d'un compartiment de craquage, celui-ci était coupé en au moins deux sections, l'une étant équipée d'un garnissage ou packing, l'autre restant libre et/ou équipée de chicanes. Une telle configuration permet le craquage d'hydrocarbures différents dans deux sections distinctes du même compartiment.

Pour l'injection de fluides gazeux ou d'hydrocarbures liquides vaporisables dans les compartiments de stripage et/ou de craquage, les moyens d'injection des fluides gazeux sont choisis préférentiellement parmi les injecteurs à cannes pulvérisant et les anneaux de dispersion.

Pour une meilleure compréhension de l'invention, les dessins annexés montrent schématiquement les dispositifs de stripage de l'art antérieur comparés à des dispositifs de l'invention, ceux-ci étant donnés à titre illustratif mais non limitatif de l'invention.

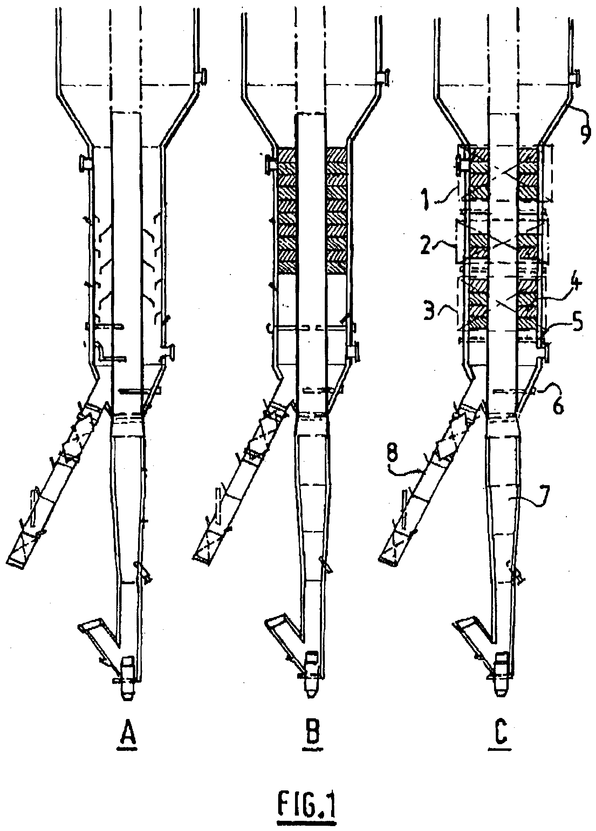

La figure 1 comprend trois modes de réalisation d'un désengageur/strippeur pour la séparation catalyseur coké/effluents de craquage issus du réacteur principal et pour le stripage, modes A, B et C :
Mode A est une unité de FCC classique dont le stripeur du désengageur/stripeur est équipé d'internes dits « disc and donuts » : cette technologie montre des limitations hydrauliques lorsque le flux de catalyseur devient trop important ou des phénomènes d'engorgement, similaires à ce qui peut se rencontrer dans des colonnes de distillation.
Mode B décrit comme A le stripeur du désengageur/stripeur mais dans lequel on a introduit des éléments structurés de technologie décrite dans les brevets cités dans la présente description : dans un arrangement classique, ces internes, permettent d'opérer à des flux de catalyseur très élevés tout en conservant une très bonne efficacité de stripage. Chaque compartiment comprend au moins un élément structuré ou « packing » et un anneau pour la distribution de gaz placé en aval du garnissage.
Mode C est un dispositif selon l'invention disposé au sein du stripeur du désengageur/stripeur.

La figure 2 détaille un mode de réalisation de C selon l'invention.

Les figures 3 et 4 détaillent un compartiment de craquage dans lequel seulement une partie de l'espace de craquage est remplie par un garnissage structuré.

Les figures 5a, 5b et 5c décrivent les différentes modifications faites sur le stripeur du désengageur/stripeur d'un pilote de craquage catalytique supportant la démonstration de l'exemple II ci-après.

Dans la figure 1, le Mode C figure le stripeur du désengageur/stripeur qui contient plusieurs compartiments. Chaque compartiment présente une fonctionnalité différente. Il y a ici 3 compartiments (1), (2) et (3) représentés dans le même stripeur. Le premier compartiment (1) dit de pré-stripage permet d'éliminer partiellement ou totalement les hydrocarbures piégés dans le catalyseur à la sortie du désengageur, un compartiment de craquage (2) dans lequel on injecte une coupe hydrocarbonée, et un compartiment (3) de stripage final, nécessaire à l'extraction les hydrocarbures piégés dans le catalyseur avant la régénération de celui-ci sous air dans le régénérateur de l'unité FCC.

Il est possible d'introduire autant de compartiments que l'on souhaite dans le même stripeur tant et pour autant que le dernier compartiment d'un tel agencement est celui du stripage final à la vapeur ou autre gaz inerte du catalyseur en vue de le préparer pour sa régénération en réduisant l'entraînement d'hydrocarbures susceptibles d'augmenter la température du régénérateur.

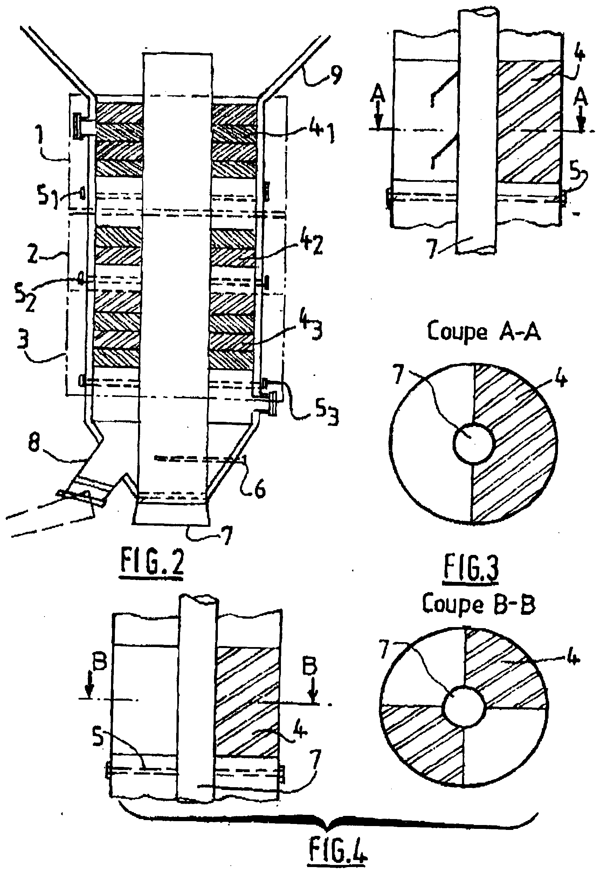

Dans la figure 2, le dispositif selon l'invention est décrit et comprend trois compartiments (1), (2) et (3) disposés autour du réacteur ascendant (7) dans le stripeur (9) d'un désengageur/stripeur d'une unité de FCC classique, avant la sortie du catalyseur (8) vers le régénérateur. Le compartiment (1) est un compartiment de pré-stripage, comprenant un garnissage structuré (4₁) comprenant un ou plusieurs étages de packing et une injection de vapeur (5₁). Le compartiment (2) est un compartiment de craquage, comprenant un garnissage structuré (4₂) comprenant un ou plusieurs étages de packing et une injection d'hydrocarbures (5₂). Le compartiment (3) est un compartiment de stripage, comprenant un garnissage structuré (4₃) comprenant un ou plusieurs étages de packing et une injection de vapeur (5₃).

Dans les figures (3) et (4), on décrit un compartiment de craquage dans lequel le garnissage (4) n'occupe qu'une partie du compartiment. Dans la figure (3), le garnissage (4) structuré n'occupe que la moitié du compartiment sur toute la hauteur de celui-ci. Dans la figure (4), il est visible que le garnissage (4) occupe deux quarts non voisins sur toute la hauteur du compartiment de craquage.

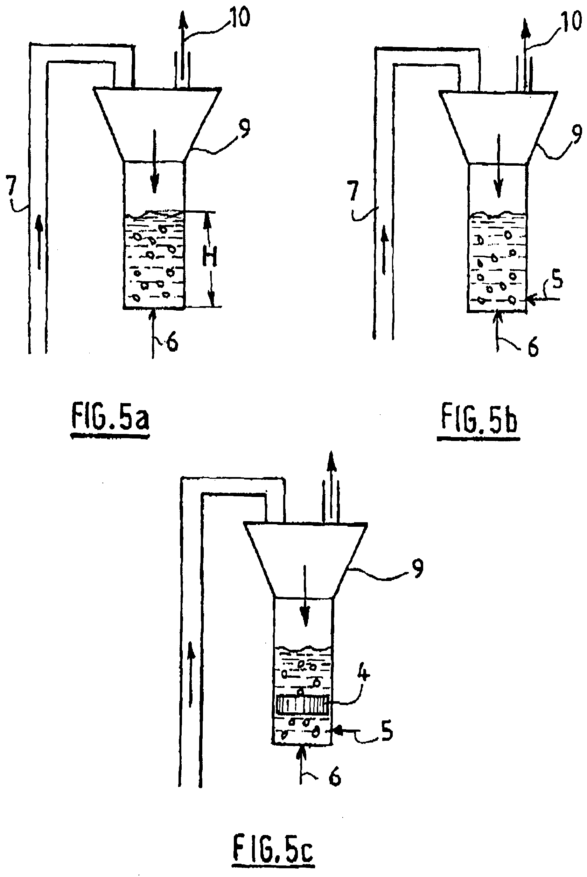

Les figures 5(a), 5(b) et 5(c) simulent le stripeur dans le désengageur-striper d'une unité de craquage catalytique : le stripeur est représenté sans recyle, à la figure 5(a), avec recycle d'hydrocarbures (5) à la figure 5 (b), et avec recycle d'hydrocarbures (5) et introduction d'un élément de garnissage (4) à la figure 5(c).

Sur l'ensemble des figures, les mêmes références désignent les mêmes éléments.

Les exemples ci-après visent à illustrer les effets de l'invention et ses avantages, mais sans en limiter la portée.

### EXEMPLE I

Le présent exemple vise à reproduire par des tests pilotes ce qui se passe dans un réacteur de craquage à lit fluidisé en vue de démontrer l'avantage de pré-stripper la catalyseur coké avec un taux de carbone résiduel non nul, en vue de restaurer une partie de son activité avant une nouvelle étape de craquage telle que revendiquée dans le cadre de la présente invention.

Un catalyseur référencé A (Conquest 85 d'Albermarle) utilisé classiquement, après régénération est introduit dans un pilote de craquage catalytique. C'est un pilote ACE-Model R+ ("Advanced Cracking Evaluation-reasearch unit with automatic catalyst addition - en français Unité avancée de craquage pour évaluation et recherche avec addition automatique de catalyseur) vendu par Kayser technologies. C'est un outil permettant le test sélectif de catalyseurs de craquage catalytique à lit fluidisé, l'évaluation des charges et le suivi technique des performances des unités industrielles.

Ce catalyseur régénéré avec un taux de carbone résiduel nul, subit une opération de craquage catalytique dans un pilote FCC en lit fluidisé. Une charge classique de type distillat sous vide (DSV) très paraffinique est injectée dans le lit fluidisé à une température de réaction de 520°C, les conditions de craquage sont telles que le ratio massique catalyseur sur charge (R) est de 5. Un premier craquage de la charge est réalisé sur le catalyseur A régénéré, un deuxième craquage est réalisé lorsque le catalyseur utilisé est celui récupéré après la première étape de craquage ; il sera référencé B. A l'issue de cette deuxième étape de craquage, le catalyseur B est strippé pour donner le catalyseur C qui sera utilisé dans une troisième étape de craquage de la même charge.

Tous les résultats sont rassemblés dans le tableau I ci-après : la première colonne donne les rendements obtenus pour le premier craquage de la charge sur le catalyseur A parfaitement régénéré, la deuxième colonne donne les rendements obtenus avec le catalyseur B (catalyseur A coké lors du premier craquage) et la troisième colonne donne ces rendements avec le catalyseur C (catalyseur A coké lors du premier craquage et ayant subit un stripage préalable).

**Tableau I**

| | **Catalyseur A** | **Catalyseur B** | **Catalyseur C** |
|---|---|---|---|
| **H2- C2** | 1.26 | 1.34 | 1.01 |
| **C3⁼** | 4.90 | 2.10 | 4.14 |
| **C3(tous)** | 5.78 | 2.50 | 4.79 |
| **iC4** | 3.71 | 0.73 | 1.81 |
| **nC4** | 0.71 | 0.21 | 0.42 |
| **iC4⁼** | 1.75 | 1.03 | 1.81 |
| **C4 (tous)** | 10.17 | 3.67 | 7.05 |
| **GPL** | 15.95 | 6.17 | 11.85 |
| **PI-160°C** | 34.94 | 15.58 | 27.65 |
| **160-221°C** | 11.57 | 7.03 | 9.59 |
| **Essence C5-221°C** | 46.51 | 22.62 | 37.25 |
| **LCO 221-360°C** | 11.07 | 14.92 | 11.95 |
| **Résidu de fond 360⁺** | 22.72 | 54.64 | 36.16 |
| **Coke** | 2.49 | 0.40 | 1.78 |
| **Delta Coke** | 0.50 | 0.08 | 0.36 |
| **Conversion -221°C** | 66.21 | 30.43 | 51.89 |
| **Conversion liquide*** | 73.53 | 43.71 | 61.04 |
| **R** | 5.00 | 5.00 | 5.00 |

| | | | |
|---|---|---|---|
| *conversion liquide = GPL+essence+LCO | | | |

On constate que la conversion standard passe de près de 44% pour le catalyseur coké non strippé (B) à 61% pour le catalyseur coké mais strippé (C). Cependant elle reste inférieure au niveau de conversion liquide atteint par le catalyseur parfaitement régénéré (A), soit ici de 73.5%. L'élimination des hydrocarbures résiduels présents dans les interstices du catalyseur ou encore dans les espaces inter-granulaires a donc permis de restaurer de manière significative l'activité catalytique du catalyseur (C) ou (A coké puis strippé).

Les résultats de ces essais montrent clairement l'intérêt de stripper le catalyseur après une première étape de craquage en vue de son utilisation pour une nouvelle étape de craquage, même si ce dernier présente une teneur en carbone ou coke résiduelle importante, ici de 0.5%.

### EXEMPLE 2

Le présent exemple vise à démontrer l'avantage qu'il y a à utiliser un interne type packing en vue d'augmenter le craquage dans le désengageur-striper d'un lit fluidisé lorsqu'on recycle un hydrocarbure en présence du dit interne, le dit interne améliorant le contact entre la phase gaz et la solide fluidisé

Des essais ont été réalisés sur un pilote fluidisé continu et adiabatique, représentatif d'une unité industrielle de craquage catalytique. Ce pilote est équipé des mêmes équipements qu'une unité industrielle. Cette unité comprend un riser au bas duquel est injectée la charge à craquer, un désengageur-striper pour séparer dans un premier temps les gaz craqués du catalyseur puis pour striper le catalyseur coké, un régénérateur pour brûler le coke présent sur le catalyseur arrivant du désengageur-striper en vue d'en restaurer l'activité avant son retour vers l'extrémité inférieure du riser. L'étape de régénération peut être faite en une ou deux étapes.

Le désengageur-striper, représenté dans la figure 5a, a été modifié pour permettre le recycle d'un hydrocarbure (référence 5 sur les figures 5b et 5c) et son craquage sur du catalyseur coké stripé ou non, en vue d'en apprécier sa craquabilité dans différentes conditions. Les figures 5a, 5b et 5c comprennent un réacteur ascendant (7) raccordé dans sa partie supérieure à un désengageur-striper (9) comprenant dans son extrémité supérieure une partie conique pour la réception du catalyseur coké et une conduite d'évacuation (10) pour l'évacuation des effluents de craquage de la charge injectée au niveau du réacteur, et dans son extrémité inférieure une partie cylindrique contenant le lit de catalyseur coké de hauteur H, comprenant un injecteur de gaz (6) pour le stripage des grains de catalyseurs cokés qui seront dirigés vers le régénérateur. Dans la figure 5b, un injecteur d'essence (5) a été rajouté pour le recycle d'essence dans le lit de catalyseur coké : dans la figure 5c, on a ajouté un élément de garnissage (4) au-dessus de l'injection d'essence (5) pour améliorer le contact essence catalyseur coké.

Les conditions opératoires sont les suivantes :
- débit charge fraîche injectée en bas du réacteur ascendant (7) =5.5kg/h
- Température de préchauffe de la charge fraîche= 200°C
- Température de réaction en haut du riser = 510°C
- Débit d'air ajusté au régénérateur pour obtenir un excès d'O₂ de 2% vol
- Débit d'azote de strippage = 450 Nl/h sans injection d'essence et de 400Nl/h en présence d'essence injectée (5) en fond du désengageur-stripper.
- L'alimentation en catalyseur est ajustée pour équilibrer le bilan thermique. Pour tous nos essais, elle est maintenue constante et voisine de 45 kg/h
- Débit d'injection d'essence (5) en fond du désengageur-stripper=1l/h
- il n'y a qu'un seul élément de garnissage type packing pour promouvoir le contact entre le gaz (6)+(5) montant dans le stripper et le solide descendant.

Le catalyseur utilisé est identique au catalyseur utilisé dans l'exemple I. Son activité catalytique MAT a été mesurée à 67% pour une surface active de 125 m²/g. Ce catalyseur ne comporte pas d'additif booster de GPL à base de ZSM5.

La charge fraîche injectée en bas du réacteur (7) est un distillat sous vide préalablement de coupe de distillation de température comprise entre 350 et 550°C. L'essence recyclée dans le stripper sous forme liquide est une essence catalytique, appelée essence de cœur, d'intervalle d'ébullition compris entre 90 et 160°C pour une masse volumique à 15°C de 752 kg/m³.

Plusieurs essais ont été réalisés dans les configurations des figures 5b et 5c et les résultats ont été rassemblés dans le tableau II ci-après. Lors de ces essais, les bilans matière sont obtenus entre 97 et 102%.

**Tableau II**

| **Configuration** | **5b** | **5c** |
|---|---|---|
| **Produit recyclé** | Essence de coeur | Essence de coeur |
| **H2** | 0.25 | 0.21 |
| **C1** | 1.38 | 1.25 |
| **H2S** | 0.01 | 0.01 |
| **C2** | 1.53 | 1.41 |
| **C2⁼** | 0.94 | 0.84 |
| **Gaz Secs H2-C2** | 4.11 | 3.72 |
| **C3** | 2.02 | 1.50 |
| **C3⁼** | 2.65 | 6.50 |
| **iC4** | 0.16 | 2.40 |
| **nC4** | 0.90 | 0.55 |
| **C4⁼¹** | 0.45 | 0.56 |
| **iC4⁼** | 1.81 | 3.10 |
| **C4⁼²** | 0.36 | 1.58 |
| **GPL** | 8.34 | 16.19 |
| **LCN (Pi-100°C)** | 14.69 | 16.7 |
| **MCN (100-150°C)** | 42.84 | 37.47 |
| **HCN (150-220°C)** | 13.03 | 11.2 |
| **TCN (i-220°C)** | 70.55 | 65.37 |
| **LCO (220-350°C)** | 8.49 | 7.24 |
| **DCO (350+°C)** | 6.91 | 5.41 |
| **Coke** | 1.60 | 2.07 |

La craquabilité de l'essence recyclée est estimée en effectuant des différences entre les bilans matière, préalablement moyennés, réalisés sans recycle et avec recycle.

En l'absence d'élément de garnissage (configuration 5b), le niveau de craquage de l'essence recyclée reste relativement faible avec une production de GPL limitée. L'introduction d'un seul élément de garnissage (configuration 5c) permet d'augmenter significativement la production de GPL et plus particulièrement celle de propylène (C3=).

Ainsi dans la configuration (5b), l'essence injectée sous forme liquide se vaporise rapidement en formant de grosses bulles qui montent très rapidement dans le lit, ce qui limite très fortement le transfert de matière entre les deux phases (catalyseur/liquide vaporisé). L'utilisation d'un élément de garnissage (configuration 5C) permet d'une part de réduire la taille des bulles mais « force » ces dernières à entrer en contact avec le solide descendant, le contact catalyseur liquide vaporisé étant ainsi plus fort.

### EXEMPLE 3

Le présent exemple vise à montrer l'impact du recycle de l'essence légère et du HCO produit dans le riser au stripeur dans le but de maximiser la production de distillat tout en conservant la production de LPG.

Les données présentées sont issues de résultats obtenus en laboratoire sur unité pilote adiabatique à lit circulant similaire à une unité industrielle sur une charge composée majoritairement de résidu atmosphérique et un catalyseur d'activité modérée (MAT ∼ 65). Ce n'est pas le même cata que l'exemple 1, sans importance. Les resultats sont donnés dans le tableau III suivant pour les différentes marches envisagées.

**Tableau III**

| Configuration | **Cas 1** | **Cas 2** | **Cas 3** |
|---|---|---|---|
| Mode | Essence | Distillat | Distillat |
| Recycle stripeur | Non | Non | Oui (LCN et HCO) |
| ROT (°C) | 520°C | 490°C | 490°C |
| H2 | 0.4 | 0.4 | 0.4 |
| C1 | 1.3 | 0.8 | 1.1 |
| C2 | 1.1 | 0.7 | 1.0 |
| C2= | 1.0 | 0.5 | 0.7 |
| Gaz secs | 3.9 | 2.5 | 3.3 |
| C3 | 0.9 | 0.7 | 1.0 |
| C3= | 3.7 | 2.0 | 3.4 |
| iC4 | 1.4 | 0.9 | 1.4 |
| nC4 | 0.5 | 0.4 | 0.5 |
| C4=1 | 1.1 | 0.6 | 0.8 |
| iC4= | 1.6 | 1.0 | 1.5 |
| C4=² | 3.4 | 1.9 | 2.3 |
| **LPG** | **11.0** | **6.6** | **9.9** |
| **LCN (Pi-150°C)** | **35.0** | **24.0** | **18.4** |
| HCN (150-220°C) | 14.0 | 13.5 | 15.9 |
| TCN (Pi-220°C) | 49.0 | 37.5 | 34.3 |
| LCO (220-350°C) | 18.3 | 23.5 | 26.6 |
| **Distillat (150-350°C)** | **32.3** | **36.9** | **42.5** |
| Slurry (350+) | 9.9 | 23.0 | 17.7 |
| Coke | 8.0 | 6.9 | 8.1 |

Dans ce tableau III, la première colonne (cas 1) montre la structure de rendement obtenue en mode essence pour une température de réaction de 520°C et un rapport catalyseur sur charge fraîche de 6.

La seconde colonne (cas 2) illustre la structure de rendement en mode distillat obtenu en adaptant les conditions opératoires, principalement la température de réaction (diminution de 520°C à 490°C), qui permet d'accroitre la production de distillat (coupe HCN /essence lourde+LCO/distillat) mais conduit à une perte en LPG et une augmentation des fractions slurry (HCO/350-440°C+DCO/440°C+). Cependant, la production de coke est également réduite ce qui permet de recycler une partie des hydrocarbures séparés par fractionnement au stripeur.

La dernière colonne (cas 3) illustre la structure de rendement après recycle au stripeur de 60% de l'essence de cœur (100-150°C) produite au réacteur principal et 50% du HCO (350-440°C) produit également au réacteur principal, l'essence étant injectée dans la première zone de craquage après le stripage préliminaire des grains séparés dans le désengageur et le HCO dans la seconde zone de craquage après stripage des grains de catalyseur issus de la première zone de craquage : ces deux zones de craquage sont équipées de « packing » ainsi que les zones de stripage, y compris dans la dernière zone avant que le catalyseur ne soit dirigé vers le régénérateur. Dans ces conditions de craquage à la fois au niveau du réacteur principal et des deux zones de craquage aménagées dans la partie stripeur du désengageur stripeur, les quantités recyclées permettent d'augmenter la production de LPG jusqu'à un volume proche de celui du mode essence (cas 1) tout en réduisant de moitié la quantité d'essence légère et en augmentant la quantité de distillat de 30% par rapport au mode essence. Le rendement coke est ainsi maintenu par rapport à celui obtenu dans un mode essence sans imposer de contrainte supplémentaire dans la zone de régénération. Le recycle permet également de diminuer de 30% la quantité de slurry (350+) produit mais ne permet cependant pas d'abaisser cette quantité jusqu'au niveau de production obtenu en mode essence. En outre, le rendement en slurry est inférieur à celui obtenu en mode distillat sans recycle vers le désengageur/stripper (cas 2).

## Revendications

1. Procédé de craquage catalytique classique à lit fluidisé comprenant :
- une étape de réaction d'une charge hydrocarbonée sur un lit fluidisé de catalyseur ascendant ou descendant,
- une étape de désengagement /stripage des grains de catalyseur cokés d'avec les effluent craqués,
- une étape de régénération, en un ou plusieurs étages, des grains de catalyseur cokés, les grains de catalyseur régénérés récupérés en sortie étant renvoyés à l'entrée du lit fluidisé de l'étape de réaction,
ledit procédé de craquage catalytique classique à lit fluidisé intégrant, dans l'étape de désengagement/stripage, un procédé de craquage et de stripage multi-étagé d'un mélange fluidisé d'hydrocarbures et de grains de catalyseur cokés comprenant au moins une étape de craquage et une étape de stripage après séparation des grains de catalyseur cokés et des effluents craqués,
**caractérisé en ce que** :
- ledit procédé de craquage catalytique classique à lit fluidisé fonctionne en mode maximisation de distillat,
- le dit procédé de craquage et de stripage multi-étagé comprend au moins deux étapes de craquage d'au moins un fluide hydrocarboné sur les grains de catalyseur cokés séparés, suivies d'au moins deux étapes de stripage de ces grains, chaque étape de craquage précédant une étape de stripage, la division des grains étant assurée dans les différentes étapes de craquage et éventuellement de stripage du procédé de craquage et de stripage multi-étagé, au moyen d'au moins un élément de garnissage structuré de type packing,
- on injecte dans la première étape de craquage au moins un hydrocarbure choisi parmi l'essence produite ou tout fluide de température d'ébullition inférieure ou égale à 220°C et dans la deuxième étape de craquage des produits avec un point d'ébullition supérieur à 350°C, de type HCO ou slurry, récupérés au niveau d'une l'étape de fractionnement des hydrocarbures du procédé de craquage catalytique à lit fluidisé.

2. Procédé selon la revendication 1, **caractérisé en ce qu'**au moins un fluide hydrocarboné est injecté sur les grains de catalyseur cokés au moyen d'injecteurs au cours de chaque étape de craquage du procédé de craquage et de stripage multi-étagé, tandis que le stripage des grains de catalyseurs cokés est effectué au cours de chaque étape de stripage du procédé de craquage et de stripage multi-étagé, par un fluide de stripage, injecté à contre-courant des grains de catalyseur utilisés à l'étape précédente.

3. Procédé selon l'une des revendications 1 ou 2, **caractérisé en ce qu'**il comprend en amont de la première étape de craquage du procédé de craquage et de stripage multi-étagé, une étape de pré-stripage du catalyseur consistant à balayer à contre-courant du flux des grains de catalyseur cokés issus de l'étape de séparation par un fluide de stripage injecté à contre-courant du dit flux.

4. Procédé selon l'une des revendications précédentes, **caractérisé en ce que** deux étapes de craquage s'intercalent entre deux étapes de stripage du procédé de craquage et de stripage multi-étagé.

5. Procédé selon l'une des revendications précédentes, **caractérisé en ce que** les étapes de stripage du procédé de craquage et de stripage multi-étagé comprennent une injection de gaz constituée majoritairement de vapeur d'eau et les étape(s) de craquage du procédé de craquage et de stripage multi-étagé, une injection d'une coupe hydrocarbonée recyclée contenant moins de 5% de vapeur d'eau.

6. Procédé selon l'une des revendications précédentes, **caractérisé en ce que** les étapes de craquage du procédé de craquage et de stripage multi-étagé comprennent une injection d'au moins un recycle d'hydrocarbures comprenant de l'essence produite ou tout fluide de température d'ébullition inférieure ou égale à 220°C et/ou des produits de type HCO ou des slurry, récupérés au niveau de l'étape de fractionnement des hydrocarbures du procédé de craquage catalytique à lit fluidisé et/ou des produits issus d'autres unités de raffinage, notamment d'unités d'hydrocraquage, de la biomasse et/ou de charges paraffiniques, avantageusement les hydrocarbures pour le recycle peuvent être choisis parmi les oléfines en C₆ et C₇ et les oligomères de température d'ébullition inférieure à 220°C résultant de la polymérisation des oléfines de C₂ à C₅.

7. Procédé selon l'une des revendications précédentes, **caractérisé en ce qu'**on injecte des hydrocarbures de même nature dans des étapes de craquage différentes du procédé de craquage et de stripage multi-étagé ou des hydrocarbures de nature différente à chacune des étapes de craquage du procédé de craquage et de stripage multi-étagé ou encore des hydrocarbures de nature différentes conjointement dans un même compartiment d'une même étape de craquage du procédé de craquage et de stripage multi-étagé.

8. Procédé selon l'une des revendications précédentes, **caractérisé en ce que** le procédé de craquage et de stripage multi-étagé comprend une succession d'étapes de craquage et de stripage, comprenant une étape initiale de pré-stripage, avantageusement, les étapes de craquage et de stripage s'intercalent les unes entre les autres, la première étape étant toujours une phase de craquage précédée d'une étape initiale de pré-stripage, la dernière une phase de stripage.

9. Procédé selon l'une des revendications précédentes, **caractérisé en ce que** le catalyseur utilisé est composé d'un catalyseur conventionnel contenant jusqu'à 15% en poids d'au moins un additif favorisant le craquage en oléfines dont au moins un additif à base de ZSM5.

10. Procédé selon l'une des revendications précédentes, **caractérisé en ce que** le procédé de craquage et de stripage multi-étagé met en œuvre un dispositif de séparation et de stripage comprenant une capacité dite désengageur/stripeur, disposée en aval d'un réacteur principal (7) contenant le lit fluidisé d'une unité de craquage catalytique à lit fluidisé, cette capacité comprenant un désengageur pour la séparation centrifuge et/ou balistique des grains de catalyseur et des hydrocarbures résultant de la réaction de craquage, et un stripeur pour le stripage des grains de catalyseur (9) disposé en amont de la sortie (8) du catalyseur, et **en ce que** le stripeur comprend au moins quatre compartiments, au moins deux compartiments de craquage (2) et au moins deux compartiments de stripage (3), comportant chacun à leur base au moins un moyen d'injection de fluides gazeux (5), équipés d'éléments de garnissage structurés ou packing, ces éléments occupant tout ou partie de la section du dit compartiment.

11. Procédé selon la revendication 10, **caractérisé en ce que** le stripeur comprend au moins un compartiment de pré-stripage (1) des grains de catalyseur situé en amont du premier compartiment de craquage, équipé d'au moins un moyen d'injection d'un de fluide de stripage, et éventuellement d'au moins un élément de garnissage structuré ou non.

12. Procédé selon l'une des revendications 10 et 11, **caractérisé en ce que** le stripeur comprend une pluralité de compartiments de craquage et de stripage intercalés, équipés d'au moins un moyen d'injection de fluides gazeux, les volumes de la totalité des compartiments de craquage et de stripage correspondant respectivement 25 à 65% et de 35 à 75% du volume de la zone de stripage.

13. Procédé selon l'une des revendications 11 et 12, **caractérisé en ce que** le compartiment de pré-stripage occupe jusqu'à 25% du volume total de stripage.

## Patentansprüche

1. Herkömmliches katalytisches Fließbett-Crackverfahren, umfassend:
- einen Reaktionsschritt eines Kohlenwasserstoff-Einsatzmaterials auf einem Fließbett eines aufsteigenden oder absteigenden Katalysators,
- einen Schritt des Auslösens / Strippens der verkokten Katalysatorkörner von dem gecrackten Abwasser,
- einen Schritt der Regeneration der verkokten Katalysatorkörner in einer oder mehreren Stufen, wobei die am Auslass zurückgewonnenen regenerierten Katalysatorkörner zum Einlass des Fließbettes des Reaktionsschritts zurückgeführt werden,
wobei das herkömmliche katalytische Fließbett-Crackverfahren in dem Schritt des Auslösens / Strippens ein mehrstufiges Crack- und Strippingverfahren eines fluidisierten Gemischs aus Kohlenwasserstoffen und verkokten Katalysatorkörnern einschließt, umfassend mindestens einen Crackschritt und einen Strippingschritt nach Trennung der verkokten Katalysatorkörner und des gecrackten Abwassers,
**dadurch gekennzeichnet, dass**:
- das herkömmliche katalytische Fließbett-Crackverfahren im Destillatmaximierungsmodus arbeitet,
- das mehrstufige Crack- und Strippingverfahren mindestens zwei Crackschritte von mindestens einem Kohlenwasserstofffluid auf den abgetrennten verkokten Katalysatorkörnern umfasst, gefolgt von mindestens zwei Strippingschritten dieser Körner, wobei jeder Crackschritt vor einem Strippingschritt erfolgt, wobei die Teilung der Körner in den verschiedenen Crack- und gegebenenfalls Strippingschritten des mehrstufigen Crack- und Strippingverfahrens mittels mindestens eines strukturierten Packungselements von Typ Packing sichergestellt wird,
- im ersten Crackschritt mindestens ein Kohlenwasserstoff, der aus dem hergestellten Benzin oder jedem Fluid mit einer Siedetemperatur von unter oder gleich 220° C ausgewählt wird, und im zweiten Crackschritt Produkte mit einem Siedepunkt von über 350 °C vom Typ HCO oder Slurry eingeleitet werden, die in einem Fraktionierungsschritt der Kohlenwasserstoffe des katalytischen Fließbett-Crackverfahrens zurückgewonnen werden.

2. Verfahren nach Anspruch 1, **dadurch gekennzeichnet, dass** bei jedem Crackschritt des mehrstufigen Crack- und Strippingverfahrens mindestens ein Kohlenwasserstofffluid mittels Injektoren auf die verkokten Katalysatorkörner geleitet wird, wohingegen das Stripping der verkokten Katalysatorkörner bei jedem Strippingschritt des mehrstufigen Crack- und Strippingverfahrens durch ein Strippingfluid durchgeführt wird, das im Gegenstrom zu den im vorhergehenden Schritt verwendeten Katalysatorkörner eingeleitet wird.

3. Verfahren nach einem der Ansprüche 1 oder 2, **dadurch gekennzeichnet, dass** es vor dem ersten Crackschritt des mehrstufigen Crack- und Strippingverfahrens einen Vorstrippingschritt des Katalysators umfasst, der darin besteht, verkokte Katalysatorkörner aus dem Trennungsschritt mit einem Strippingfluid , das entgegen dem Strom eingeleitet wird, entgegen dem Strom zu überstreichen.

4. Verfahren nach einem der vorhergehenden Ansprüche, **dadurch gekennzeichnet, dass** zwischen zwei Crackschritten des mehrstufigen Crack- und Strippingverfahrens zwei Crackschritte eingefügt werden.

5. Verfahren nach einem der vorhergehenden Ansprüche, **dadurch gekennzeichnet, dass** die Strippingschritte des mehrstufigen Crack- und Strippingverfahrens ein Einleiten von Gas umfassen, bestehend hauptsächlich aus Wasserdampf, und der/die Crackschritt/e des mehrstufigen Crack- und Strippingverfahrens ein Einleiten einer recycelten Kohlenwasserstofffraktion, die weniger als 5 % Wasserdampf enthält.

6. Verfahren nach einem der vorhergehenden Ansprüche, **dadurch gekennzeichnet, dass** die Crackschritte des mehrstufigen Crack- und Strippingverfahrens ein Einleiten von mindestens einem Recycling von Kohlenwasserstoffen umfassen, umfassend hergestelltes Benzin oder jedes Fluid mit einer Siedetemperatur von unter oder gleich 220 °C und/oder Produkte vom Typ HCO oder Slurry, zurückgewonnen beim Fraktionierungsschritt der Kohlenwasserstoffe des katalytischen Fließbett-Crackverfahrens, und/oder Produkte aus anderen Raffinerieeinheiten, insbesondere Hydrocrackeinheiten, Biomasse und/oder Paraffin-Einsatzmaterialien, wobei die Kohlenwasserstoffe für das Recycling aus den C₆- und C₇-Olefinen und den Oligomeren mit einer Siedetemperatur von unter 220 °C als Ergebnis der Polymerisation der C₂- bis C₅-Olefine ausgewählt sein können.

7. Verfahren nach einem der vorhergehenden Ansprüche, **dadurch gekennzeichnet, dass** bei unterschiedlichen schritten des mehrstufigen Crack- und Strippingverfahrens gleichartige Kohlenwasserstoffe oder verschiedenartige Kohlenwasserstoffe bei jedem der Crackschritte des mehrstufigen Crack- und Strippingverfahrens oder auch verschiedenartige Kohlenwasserstoffen zusammen ein gleiches Abteil des gleichen Crackschritts des mehrstufigen Crack- und Strippingverfahrens eingeleitet werden.

8. Verfahren nach einem der vorhergehenden Ansprüche, **dadurch gekennzeichnet, dass** das mehrstufige Crack- und Strippingverfahrens eine Abfolge von Crack- und Strippingschritten umfasst, umfassend einen einleitenden Vorstrippingschritt, wobei sich die Crack- und Strippingschritte ineinander verschachteln, wobei der erste Schritt immer eine Crackphase ist, der ein einleitender Vorstrippingschritt vorausgeht, der letzte eine Strippingphase.

9. Verfahren nach einem der vorhergehenden Ansprüche, **dadurch gekennzeichnet, dass** der verwendete Katalysator aus einem üblichen Katalysator besteht, der zu bis zu 15 Gew.-% mindestens eines das Cracken in Olefine fördernden Zusatzstoffs enthält, davon mindestens einen Zusatzstoff auf der Basis von ZSM5.

10. Verfahren nach einem der vorhergehenden Ansprüche, **dadurch gekennzeichnet, dass** für das mehrstufige Crack- und Strippingverfahren eine Trenn- und Strippingvorrichtung verwendet wird, die eine sogenannte Auslösung-/Strippingkapazität verwendet, die nach einem Hauptreaktor (7) angeordnet ist, der das Fließbett einer katalytischen Fließbett-Crackanlage enthält, wobei diese Kapazität einen Auslöser für die zentrifugale und oder ballistische Trennung der Katalysatorkörner und der Kohlenwasserstoffe im Ergebnis der Crackreaktion umfasst, und einen Stripper für das Stripping der Katalysatorkörner (9), die vor dem Auslass (8) des Katalysators angeordnet sind, und dadurch, dass der Stripper mindestens vier Abteile umfasst, mindestens zwei Crackabteile (2) und mindestens zwei Strippingabteile (3), die an ihrer Basis jeweils mindestens ein Mittel zum Einleiten von gasförmigen Fluiden (5) aufweisen, ausgestattet mit strukturierten Packungselementen oder Packing, wobei diese Elemente den Querschnitt des Abteils ganz oder teilweise belegen.

11. Verfahren nach Anspruch 10, **dadurch gekennzeichnet, dass** der Stripper mindestens ein Vorstrippingabteil (1) der Katalysatorkörner umfasst, das sich vor dem ersten Crackabteil befindet, ausgestattet mit mindestens einem Mittel zum Einleiten eines Strippingfluids und gegebenenfalls mit mindestens einem strukturierten oder nicht strukturierten Packungselement.

12. Verfahren nach einem der Ansprüche 10 und 11, **dadurch gekennzeichnet, dass** der Stripping eine Vielzahl verschachtelter Crack- und Strippingkammern umfasst, die mit mindestens einem Mittel zum Einleiten gasförmiger Fluide ausgestattet sind, wobei die Volumina aller Crack- und Strippingabteile jeweils 25 bis 65 % und 35 bis 75 % des Volumens der Strippingzone betragen.

13. Verfahren nach einem der Ansprüche 11 und 12, **dadurch gekennzeichnet, dass** das Vorstrippingabteil bis zu 25 % des gesamten Strippingvolumens einnimmt.

## Claims

1. A conventional fluidized-bed catalytic cracking process comprising:
- a step of reaction of a hydrocarbon feedstock over a fluidized bed of ascending or descending catalyst,
- a step of disengaging/stripping the coked catalyst particles from cracked effluents,
- a step of regenerating, in one or more stages, the coked catalyst particles, the regenerated catalyst particles recovered at an outlet being returned to the inlet of the fluidized bed of the reaction step,
said conventional fluidized-bed catalytic cracking process incorporating, in the disengaging/stripping step, a multi-stage cracking and stripping process of a fluidized mixture of hydrocarbons and coked catalyst particles comprising at least one step of cracking and one stop of stripping after separation of the coked catalyst particles and the cracked effluents,
**characterized in that**:
- said conventional fluidized-bed catalytic cracking process operates in a mode for maximizing distillates,
- said multi-stage cracking and stripping process comprises at least two steps of cracking at least one hydrocarbon fluid over the separated coked catalyst particles, followed by at least two stripping steps of said particles, each cracking step preceding a stripping step, the division of the particles being ensured in the various cracking and optionally stripping steps of the multi-stage cracking and stripping process, by means of at least one structured packing element,
- in the first cracking step, at least one hydrocarbon chosen from the petrol produced or any fluid having a boiling point of less than or equal to 220°C is injected and, in the second cracking step, products having a boiling point higher than 350°C, of HCO type or slurry, recovered at a hydrocarbon fractioning step of the fluidized-bed catalytic cracking process.

2. The process according to claim 1, **characterized in that** at least one hydrocarbon fluid is injected over the coked catalyst particles by means of injectors during each cracking step of the multi-stage cracking and stripping process, while the stripping of the coked catalyst particles is carried out during each stripping step of the multi-stage cracking and stripping process by a stripping fluid, injected counter-currently to the catalyst particles used in the preceding step.

3. The process according to one of claims 1 or 2, **characterized in that** it comprises, upstream of the first cracking step of the multi-stage cracking and stripping process, a step of pre-stripping the catalyst that consists in counter-currently flushing the stream of coked catalyst particles, resulting from the separation step, with a stripping fluid injected counter-currently to said stream.

4. The process according to any one of the preceding claims, **characterized in that** two cracking steps are intercalated between two stripping steps of the multi-stage cracking and stripping process.

5. The process according to any one of the preceding claims, **characterized in that** the stripping steps of the multi-stage cracking and stripping process comprise a gas injection consisting predominantly of steam and the cracking step(s) of the multi-stage cracking and stripping process comprise an injection of a recycled hydrocarbon fraction containing less than 5% steam.

6. The process according to any one of the preceding claims, **characterized in that** the cracking steps of the multi-stage cracking and stripping process comprise an injection of at least one recycle of hydrocarbon comprising the produced petrol or any fluid having a boiling point less than or equal to 220°C and/or products of HCO type or slurries, recovered at the hydrocarbon fractioning step of the fluidized-bed catalytic cracking process and/or products resulting from other refinery units, in particular hydrocracking units, biomass units and/or paraffinic feedstock units, advantageously the hydrocarbons for recycle can be chosen from C₆ and C₇ olefins and oligomers having a boiling point of less than 220°C resulting from the polymerization of C₂ to C₅ olefins.

7. The process according to any one of the preceding claims, **characterized in that** hydrocarbons of the same nature are injected into different cracking steps of the multi-stage cracking and stripping process or different types of hydrocarbons are injected at each of the cracking steps of the multi-stage cracking and stripping process or even different types of hydrocarbons are injected jointly into the same compartment of the same cracking step of the multi-stage cracking and stripping process.

8. The process according to any one of the preceding claims, **characterized in that** the multi-stage cracking and stripping process comprises a sequence of cracking and stripping steps, comprising an initial pre-stripping step, advantageously, the cracking and stripping steps are intercalated between one another, the first step always being a cracking phase preceded by an initial pre-stripping step, the last being a stripping phase.

9. The process according to any one of the preceding claims, **characterized in that** the catalyst used is composed of a conventional catalyst containing up to 15% by weight of at least one additive that promotes the cracking of olefins, of which at least one additive is based on ZSM5.

10. The process according to any one of the preceding claims, **characterized in that** the multi-stage cracking and stripping process uses a separation and stripping device comprising a so-called disengager/stripper vessel positioned downstream of a main reactor (7) containing the fluidized bed of a fluidized-bed catalytic cracking unit, said vessel comprising a disengager for the centrifugal and/or ballistic separation of the catalyst particles and of hydrocarbons resulting from the cracking reaction, and a stripper for stripping the catalyst particles (9) which is positioned upstream of the catalyst outlet (8), and **in that** the stripper comprises at least four compartments, at least two cracking compartments (2) and at least two stripping compartments (3), each comprising at their base at least one means of injecting gaseous fluids (5), equipped with structured packing elements, these elements occupying all or some of the cross-section of said compartment.

11. The process according to claim 10, **characterized in that** the stripper comprises at least one pre-stripping compartment (1) for pre-stripping the catalyst particles located upstream of the first cracking compartment, equipped with at least one means of injecting a stripping fluid, and optionally with at least one structured or non-structured packing element.

12. The process according to any one of claims 10 and 11, **characterized in that** the stripper comprises a plurality of intercalated cracking and stripping compartments, equipped with at least one means for injecting gaseous fluids, the volumes of all of the cracking and stripping compartments corresponding respectively to 25 to 65% and 35 to 75% of the volume of the stripping zone.

13. The process according to any one of claims 11 and 12, **characterized in that** the pre-stripping compartment occupies up to 25% of the total stripping volume.
